# EUROPÄISCHE PATENTANMELDUNG

(11) **EP 2 158 922 A1**
(43) Veröffentlichungstag der Anmeldung: **03.03.2010**
(21) Anmeldenummer: 08163199.6
(22) Anmeldetag: 28.08.2008
(51) Int. Cl.: A61K 47/34, C08L 39/00

(54) **Verfahren zur Trocknung von als Solubilisatoren für in Wasser schwerlösliche Verbindungen geeigneten Copolymeren auf Basis von Polyethern**

(71) Anmelder: BASF SE, 67056 Ludwigshafen (DE)
(72) Erfinder: Mertoglu, Murat, 67059, Ludwigshafen (DE); Dobrawa, Rainer, 68167, Mannheim (DE); Meyer-Böhm, Kathrin, 90537, Feucht (DE); Schönherr, Michael, 67227, Frankenthal (DE)

(57) **Zusammenfassung**

Verwendung von Copolymeren, erhalten durch radikalisch initiierte Polymerisation einer Mischung aus
i) 30 bis 80 Gew.-% N-Vinyllactam,
ii) 10 bis 50 Gew.-% Vinylacetat, und
iii) 10 bis 50 Gew.-% eines Polyethers,

mit der Maßgabe, dass die Summe der Komponenten i), ii) und iii) gleich 100 Gew.-% ist, als Solubilisatoren für in Wasser schwerlösliche Substanzen.

## Beschreibung

Die vorliegende Erfindung betrifft ein Verfahren zur Trocknung von Copolymeren, die durch Polymerisation von Vinylacetat und N-Vinyllactamen in Gegenwart eines Polyethers in Lösung erhalten werden. Solche Copolymeren eignen sich als Solubilisatoren für in Wasser schwerlösliche Substanzen.

Aus der WO 2007/051743 ist die Herstellung und Verwendung von von Copolymeren, die durch Polymerisation von Vinylacetat und N-Vinyllactamen in Gegenwart eines Polyethers in Lösung erhalten werden und deren Verwendung als Solubilisatoren für in Wasser schwerlösliche Substanzen beschrieben. Es ist auch beschrieben, dass die Copolymeren unter anderem durch Sprühtrocknung getrocknet werden können. Jedoch hat neigen die sprühgetrockneten Produkte zur Verblockung.

Aufgabe der vorliegenden Erfindung war ein verbessertes Verfahren zur Sprühtrocknung von Copolymeren, die durch Polymerisation von Vinylacetat und N-Vinyllactamen in Gegenwart eines Polyethers in Lösung erhalten werden

Die Aufgabe wurde erfindungsgemäß gelöst durch ein Verfahren zur Sprühtrocknung von wasserlöslichen oder wasserdispergierbaren Copolymerisaten, die erhalten werden durch radikalisch initiierte Polymerisation in Lösung einer Mischung aus
i) 30 bis 80 Gew.-% N-Vinyllactam,
ii) 10 bis 50 Gew.-% Vinylacetat und
iii) 10 bis 50 Gew.-% eines Polyethers,
mit der Maßgabe, dass die Summe von i), ii) und iii) gleich 100 Gew.-% ist, welches dadurch gekennzeichnet ist, dass die Sprühtrocknung in Gegenwart eines Puderungsmittels erfolgt.

Bevorzugt werden Polymerisate aus
i) 40 bis 60 Gew.-% N-Vinyllactam
ii) 15 bis 35 Gew.-% Vinylacetat
iii) 10 bis 30 Gew.-% eines Polyethers,
nach dem erfindungsgemäßen Verfahren hergestellt

Auch für die bevorzugten Zusammensetzungen gilt die Maßgabe, dass die Summe der Komponenten i), ii), und iii) gleich 100 Gew.-% beträgt.

Als N-Vinyllactam kommen N-Vinylcaprolactam oder N-Vinylpyrrolidon oder deren Mischungen in Betracht. Bevorzugt wird N-Vinylcaprolactam verwendet.

Als Polyether kommen vorzugsweise Polyalkylenglykole in Betracht. Die Polyalkylenglykole können Molekulargewichte von 1000 bis 100000 D [Dalton], vorzugsweise 1500 bis 35000 D, besonders bevorzugt 1500 bis 10000 D, aufweisen. Die Molekulargewichte werden ausgehend von der gemäß DIN 53240 gemessenen OH-Zahl bestimmt.

Als besonders bevorzugte Polyalkylenglykole kommen Polyethylenglykole in Betracht. Weiterhin eignen sich auch Polypropylenglykole, Poly-Tetrahydrofurane oder Polybutylenglykole, die aus 2-Ethyloxiran oder 2,3-Dimethyloxiran erhalten werden..

Geeignete Polyether sind auch statistische oder blockartige Copolymere von aus Ethylenoxid, Propylenoxid und Butylenoxiden gewonnenen Polyalkylenglykolen wie beispielsweise Polyethylenglykol-polypropylenglykol-Blockcopolymere. Die Blockcopolymere können vom AB- oder vom ABA-Typ sein.

Zu den bevorzugten Polyalkylenglykolen gehören auch solche, die an einer oder an beiden OH-Endgruppen alkyliert sind. Als Alkylreste kommen verzeigte oder unverzweigte C₁-bis C₂₂-Alkylreste in Betracht, bevorzugt C₁-C₁₈-Alkylreste, beispielsweise Methyl-, Ethyl-, n-Butyl-, Isobutyl-, Pentyl-, Hexyl-, Octyl-, Nonyl-, Decyl-, Dodecyl-, Tridecyl- oder Octadecyl-Reste.
Ganz besonders bevorzugte Copolymere werden durch Polymerisation von Vinylacetat und N-Vinylcaprolactam in Gegenwart von Polyethylenglykolen mit einem Molekulargewicht von 3500 bis 15000 Dalton, insbesondere 6000 Dalton erhalten.

Allgemeine Verfahren zur Herstellung der erfindungsgemäßen Copolymerisate sind an sich bekannt. Die Herstellung erfolgt durch radikalisch initiierte Polymerisation, bevorzugt Lösungspolymerisation, in nichtwässrigen, organischen Lösungsmitteln oder in gemischt nichtwässrigen/wässrigen Lösungsmitteln. Die Herstellung der Copolymerisate ist in der WO 2007/051743, auf deren Offenbarung hiermit ausdrücklich Bezug genommen wird, ausführlich beschrieben.

Die Copolymerisate werden als Lösungen in organischen Lösungsmitteln, Mischungen aus Wasser und organischen Lösungsmitteln oder als wässrige Dispersionen erhalten. Geeignete nichtwässrige organische Lösungsmittel sind beispielsweise Alkohole, wie Methanol, Ethanol, n-Propanol, und Isopropanol sowie Glykole, wie Ethylenglykol und Glycerin oder Ester wie beispielsweise Ethylacetat, n-Propylacetat, Isopropylacetat, Isobutylacetat oder Butylacetat.

Der Feststoffgehalt der erhaltenen wässrigen Polymerisat-Dispersionen oder Lösungen beträgt in der Regel 10 bis 70 Gew.-%, bevorzugt 10 bis 60 Gew.-%, besonders bevorzugt 10 bis 40 Gew.-%, insbesondere 10 bis 20 Gew.-%..

Die Polymerisat-Dispersionen oder Lösungen weden erfindungsgemäß in Gegenwart von Puderungsmitteln in Pulverform oder in Granulate überführt werden. Geeignete Puderungsmittel sind beispielweise hochdisperse Kieselsäure oder hydrophob modifizierte hochdisperse Kieselsäure, Stärke, Talkum, Kaolin, Zinkoxid oder Titandioxid.. Bevorzugt werden hochdisperse Kieselsäuren zugegeben. Die Puderungsmittel können in Mengen von 0.05 bis 5 Gew.-%, bevorzugt 0.5 bis 2 Gew.-%, bezogen auf den Feststoffgehalt der Polymerlösungen, zugegeben werden. Das Puderungsmittel kann den Polymerlösungen oder Dispersionen Im Batch vor der Einleitung in die Sprühvorrichtung zugegeben werden oder kontinuierlich vor der Versprühung zudosiert werden.

Die Sprühtrocknung kann in den üblichen kommerziell erhältlichen Sprühvorrichtungen durchgeführt werden. Die Zerstäubung oder Versprühung kann mit Einstoffdüsen, Mehrstoffdüsen, insbesondere Zweistoffdüsen, oder mittels Scheibenzerstäubung erfolgen.

Die Zulufttemperatur des Trockengases kann 50 bis 220 °C betragen, bei wässrigen Systemen bevorzugt 120 bis 160 °C. Die Ablufttemperatur kann 50 bis 80°C betragen, bevorzugt 50 bis 75 °C.

Als Trockengas kann Luft oder ein inertes Gas wie Stickstoff verwendet werden. Das Trockengas wird bevorzugt im Gegenstrom geführt. Das Trockengas kann auch nach der Kreisgasfahrweise zu- und abgeführt werden.

Der Austrag aus der Sprühvorrichtung kann auf an sich bekannte Weise beispielsweise über Förderschnecken erfolgen. Üblicherweise erfolgt der Austrag am unteren Ende der Sprühvorrichtung.

Die Copolymerisate werden als als riesel- und fließfähige wasserdispergierbare oder wasserlösliche Pulver gewonnen. Die Pulver lassen sich durch Extrusion verarbeiten. Üblicherweise liegt die Restfeuchte im Bereich von 5 Gew.-%.

Die Polymerisate weisen K-Werte nach Fikentscher im Bereich von 10 bis 60, vorzugsweise 15 bis 40, gemessen in einer 1 gew.-%igen ethanolischen Lösung, auf.

Die Versprühung kann auch in Gegenwart eines Wirkstoffs erfolgen, um direkt eine feste Lösung des Wirkstoffs im Polymer zu erzeugen.

### Anwendungen:

Die erfindungsgemäß zu verwendenden Copolymere lassen sich grundsätzlich auf allen Gebieten einsetzen, bei denen in Wasser nur schwerlösliche oder unlösliche Substanzen entweder in wässrigen Zubereitungen zum Einsatz kommen sollen oder ihre Wirkung in wässrigem Milieu entfalten sollen. Die Copolymere finden demgemäß Verwendung als Solubilisatoren von in Wasser schwerlöslichen Substanzen, insbesondere biologisch aktiven Substanzen.

Der Begriff "in Wasser schwerlöslich" umfasst erfindungsgemäß auch praktisch unlösliche Substanzen und bedeutet, dass für eine Lösung der Substanz in Wasser bei 20 °C mindestens 30 bis 100 g Wasser pro g Substanz benötigt wird. Bei praktisch unlöslichen Substanzen werden mindestens 10.000 g Wasser pro g Substanz benötigt.

Im Sinne der vorliegenden Erfindung sind unter in Wasser schwerlösliche biologisch aktive Substanzen pharmazeutische Wirkstoffe für Mensch und Tier, kosmetische oder agrochemische Wirkstoffe oder Nahrungsergänzungsmittel oder diätetische Wirkstoffe zu verstehen.

Weiterhin kommen als zu solubilisierende schwerlösliche Substanzen auch Farbstoffe wie anorganische oder organische Pigmente in Betracht.

In den folgenden Beispielen wird die Herstellung und Verwendung der erfindungsgemäßen Copolymere näher erläutert.

### Herstellung der Copolymerisate

Wie in der WO 2007/051743 beschrieben wurden Copolymere der Zusammensetzung laut Tabelle I hergestellt.

### Verwendete Abkürzungen:

VCap: N-Vinylcaprolactam
VAc: Vinylacetat
PEG: Polyethylenglykol

**Tabelle I**

| Polymer | Zusammensetzung in Gew.-% | | | K-Wert (1gew.-%iginEthanol) |
|---|---|---|---|---|
| | PEG 6000 | VCap | VAc | |
| A | 15 | 50 | 35 | 32.2 |
| B | 13 | 57 | 30 | 19,8 |

Die Polymerlösungen der Polymeren A und B wurden wie folgt in den pulverförmigen Zustand überführt:
Trockenturm: Durchmesser 80 cm, Höhe 11,5 m
Trocknungsgas: Stickstoff
Trocknungsgasdurchsatz: 480 kg/h

Die Gew.-% Angaben für das Additiv beziehen sich auf den Feststoffgehalt an Polymer.

### Besipiel 1

| | |
|---|---|
| Zerstäuber: | Einstoffdüse: 0.8 mm |
| Feststoffgealt: | 15 gew.-%ige wässrige Lösung Polymer B |
| Additiv: | 1 Gew.-% Aerosil 200 |
| Eingangstemp.: | 120°C |
| Ausgangstemp.: | 60°C |

### Beispiel 2

| | |
|---|---|
| Zerstäuber: | Zweistoffdüse, 1,3 mm Teflon |
| Additiv: | 0.9 Gew.-% Aerosil 200Feststoffgehalt: 15 Gew.-% ige wässrige Lösung Polymer A |
| Eingangstemp.: | 120°C |
| Ausgangstemp.: | 55°C |

### Beispiel 3

| | |
|---|---|
| Zerstäuber: | Zweistoffdüse, 3 mm Teflon |
| Additiv: | 1 Gew.-% Aerosil 200 |
| Feststoffgehalt: | 20 Gew.-% ige wässrige Lösung Polymer A |
| Eingangstemp.: | a) 120 °C, b) 140 °C |
| Ausgangstemp.: | a) 60 °C, b) 60 °C |

## Patentansprüche

1. Verwendung von Copolymeren, erhalten durch radikalisch initiierte Polymerisation einer Mischung aus
i) 30 bis 80 Gew.-% N-Vinyllactam,
ii) 10 bis 50 Gew.-% Vinylacetat, und
iii) 10 bis 50 Gew.-% eines Polyethers,
mit der Maßgabe, dass die Summe der Komponenten i), ii) und iii) gleich 100 Gew.-% ist,
als Solubilisatoren für in Wasser schwerlösliche Substanzen.

2. Verwendung nach Anspruch 1, wobei die Copolymere aus
i) 30 bis 70 Gew.-% N-Vinyllactam,
ii) 15 bis 35 Gew.-% Vinylacetat, und
iii) 10 bis 35 Gew.-% eines Polyethers,
erhalten werden.

3. Verwendung nach Anspruch 1 oder 2, wobei als Komponente i) N-Vinylpyrrolidon oder N-Vinylcaprolactam oder Mischungen davon eingesetzt werden.

4. Verwendung nach einem der Ansprüchen 1 bis 3, wobei wobei als Komponente i) N-Vinylcaprolactam eingesetzt wird.

5. Verwendung nach einem der Ansprüche 1 bis 4, wobei die Copolymeren. als Komponente ii) ein Polyethylenglykol enthalten.

6. Verwendung nach einem der Ansprüche 1 bis 5, wobei die Copolymeren als Komponente ii) ein Polyethylenglykol mit einem Molekulargewicht von 1000 Dalton bis 10.000 Dalton enthalten.

7. Verwendung nach einem der Ansprüche 1 bis 6 wobei die Copolymeren einen K-Wert von 10 bis 60 aufweisen.

8. Verwendung nach einem der Ansprüche 1 bis 6 wobei die Copolymeren einen K-Wert von 15 bis 40 aufweisen.

9. Verwendung nach einem der Ansprüche 1 bis 8, wobei es sich bei den in Wasser schwerlöslichen Substanzen um biologisch aktive Substanzen handelt.

10. Verwendung nach einem der Ansprüche 1 bis 9, zur Herstellung von pharmazeutischen Zubereitungen für die Behandlung von Krankheiten.

11. Verwendung nach einem der Ansprüche 1 bis 8 für kosmetische Zubereitungen.

12. Verwendung nach einem der Ansprüche 1 bis 8 für agrochemische Zubereitungen.

13. Verwendung nach einem der Ansprüche 1 bis 8 für Nahrungsergänzungsmittel oder dietätische Mittel.

14. Verwendung nach einem der Ansprüche 1 bis 8 für Lebensmittel.

15. Verwendung nach einem der Ansprüche 1 bis 8 für Zubereitungen von Farbstoffen.

16. Zubereitungen von in Wasser schwerlöslichen Substanzen, enthaltend als Solubilisatoren Copolymere, erhalten durch radikalisch initiierte Polymerisation einer Mischung aus
i) 30 bis 80 Gew.-% N-Vinyllactam,
ii) 10 bis 50 Gew.-% Vinylacetat, und
iii) 10 bis 50 Gew.-% eines Polyethers,
mit der Maßgabe, dass die Summe der Komponenten i), ii) und iii) gleich 100 Gew.-% ist.

17. Zubereitungen nach Anspruch 16, in denen die in Wasser schwerlösliche Substanz in Form einer festen Lösung in den Copolymeren vorliegen.

18. Zubereitungen nach Anspruch 16 oder 17, enthaltend als in Wasser schwerlösliche Substanz eine biologisch aktive Substanz.

19. Zubereitungen nach einem der Ansprüche 16 bis 18, enthaltend als in Wasser schwerlösliche biologisch aktive Substanz einen pharmazeutischen Wirkstoff.

20. Zubereitungen nach Anspruch 19, in Form oral applizierbarer Darreichungsformen.

21. Zubereitungen nach einem der Ansprüche 1 bis 18, enthaltend als in Wasser schwerlösliche biologisch aktive Substanz einen kosmetischen Wirkstoff.

22. Zubereitungen nach einem der Ansprüche 16 bis 18, enthaltend als in Wasser schwerlösliche biologisch aktive Substanz einen agrochemischen Wirkstoff.

23. Zubereitungen nach einem der Ansprüche 16 bis 18, enthaltend als in Wasser schwerlösliche biologisch aktive Substanz ein Nahrungsergänzungsmittel oder einen dietätischen Wirkstoff.

24. Zubereitungen nach Anspruch 16 oder 17, enthaltend als in Wasser schwerlösliche Substanz einen Farbstoff.
